# EUROPEAN PATENT APPLICATION

(11) **EP 1 623 697 A1**
(43) Date of publication of application: **08.02.2006**
(21) Application number: 04732808.3
(22) Date of filing: 13.05.2004
(51) Int. Cl.: A61K 8/18

(54) **OIL-IN-WATER EMULSION COSMETICS**

(30) Priority: 13.05.2003 JP 2003135182
(71) Applicant: The Nisshin OilliO Group, Ltd., Tokyo 104-8285 (JP)
(72) Inventor: GOTOU, N, The Nisshin OilliO Group Ltd., Yokohama-shi, Kanagawa 225-8 (JP); EHARA, T, The Nisshin OilliO Group Ltd., Yokohama-shi, Kanagawa 225-8 (JP); MORI, Takahiro, The Nisshin OilliO Group Ltd., Yokohama-shi, Kanagawa 225-8 (JP)
(74) Representative: Zimmermann, Gerd Heinrich
(86) International application number: PCT/JP2004/006806
(87) International publication number: WO 2004/100918

(57) **Abstract**

The present invention provides an oil-in-water emulsion type cosmetic preparation containing (A) at least one selected from the group consisting of ester compounds which are reaction products of ditrimethylolpropane with fatty acid, polycondensates of ditrimethylolpropane with polycarboxylic acid, polycondensates of the ester compound with polycarboxylic acid, polycondensates of fatty acid with the polycondensate of ditrimethylolpropane with polycarboxylic acid, and polycondensates of ditrimethylolpropane, fatty acid and polycarboxylic acid; (B) a nonionic surfactant with an HLB of 10 or more and/or an ionic surfactant with an HLB of 10 or more; and (C) a water-based ingredient, the component (A) having a hydroxyl value (OHV) ranging from 10 to 150. The oil-in-water emulsion type cosmetic preparation is **characterized in that** the skin feel (non-sticky feel and long-lasting moisturizing feel) is satisfactory and the long-term emulsion stability is also excellent.

## Description

### Background of the Invention

The present invention relates to an oil-in-water emulsion type cosmetic preparation, and more particularly to an oil-in-water emulsion type cosmetic preparation that can meet both requirements of a non-sticky feel and a long-lasting moisturizing feel and exhibit excellent emulsion stability.

Conventionally, the oil-in-water emulsion type cosmetic preparation has an effect of controlling the balance between the water content and the oily content in the skin, when applied to the skin, to keep the skin in good condition by the action of ingredients blended in the emulsion, such as an aqueous ingredient, an oily base, a moisturizing agent, an essence and the like. Extensive investigations have been made to additionally obtain the benefits with respect to the preservation stability, skin feel, appearance and the like by selecting the ingredients to be blended into the oil-in-water emulsion type cosmetic preparation (as disclosed in, for example, Japanese Patent No. 2657219 and Japanese Patent Unexamined Publication (JP Kokai) No. 2001-39827 and No. 2001-354510).

Although various benefits have been hitherto discussed, the benefit for moisturizing feel from the oil-in-water emulsion type cosmetic preparation is poorer than that from other forms containing an oily base, for example, water-in-oil emulsion type cosmetic preparation and oil-based cosmetic preparation. Therefore, there is an increasing demand for development of an oil-in-water emulsion type cosmetic preparation that can keep giving a non-sticky feel, which is one of the features peculiar to the oil-in-water-emulsion type cosmetic preparation, and at the same time, spread smoothly when applied to ensure the ease of use, offer a long-lasting moisturizing feel and exhibit excellent emulsion stability for an extended period of time.

### Disclosure of Invention

The inventors of the present invention have studied intensively to solve the above-mentioned problems. As a result of the study, it has been found that an oil-in-water emulsion type cosmetic preparation which can offer improved long-lasting moisturizing feel, while maintaining non-sticky feel and excellent usability and exhibit excellent emulsification of oily base to improve the long-term stability, by blending a particular ditrimethylolpropane derivative, a nonionic surfactant and/or ionic surfactant with an HLB of 10 or more, and a water-based ingredient into the formulation. The present invention has been thus accomplished.

Namely, the present invention provides an oil-in-water emulsion type cosmetic preparation comprising:
(A) at least one selected from the group consisting of ester compounds represented by the following formula (I) which are reaction products of ditrimethylolpropane with fatty acid, polycondensates of ditrimethylolpropane with polycarboxylic acid, polycondensates of the ester compound of formula (I) with polycarboxylic acid, polycondensates of fatty acid with the polycondensate of ditrimethylolpropane with polycarboxylic acid, and polycondensates of ditrimethylolpropane, fatty acid and polycarboxylic acid, 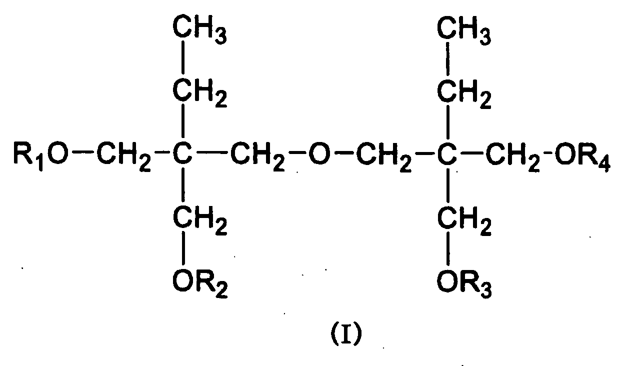
   wherein R₁ to R₄ are each independently hydrogen atom or a fatty acid residue, provided that at least one of R₁ to R₄ represents a fatty acid residue, the component (A) having a hydroxyl value (OHV) ranging from 10 to 150;
(B) a nonionic surfactant with an HLB of 10 or more and/or an ionic surfactant with an HLB of 10 or more; and
(C) a water-based ingredient.

The above-mentioned polycondensates of fatty acid with the polycondensate of ditrimethylolpropane with polycarboxylic acid indicates polycondensates obtainable by subjecting a polycondensate of ditrimethylolpropane with a polycarboxylic acid and a fatty acid to an esterification reaction.

Also, the present invention provides an oil-in-water emulsion type cosmetic preparation comprising the component (A) in an amount of 0.1 to 30% by mass, the component (B) in an amount of 0.01 to 10% by mass and the component (C) in an amount of 40 to 95% by mass.

### Best Mode for Carrying out the Invention

The component (A) for use in the oil-in-water emulsion type cosmetic preparation according to the present invention includes at least one selected from the group consisting of ester compounds represented by the following formula (I) which are reaction products of ditrimethylolpropane with fatty acid, polycondensates of ditrimethylolpropane with polycarboxylic acid, polycondensates of the ester compound of formula (I) with polycarboxylic acid, polycondensates of fatty acid with the polycondensate of ditrimethylolpropane with polycarboxylic acid, and polycondensates of ditrimethylolpropane, fatty acid and polycarboxylic acid, 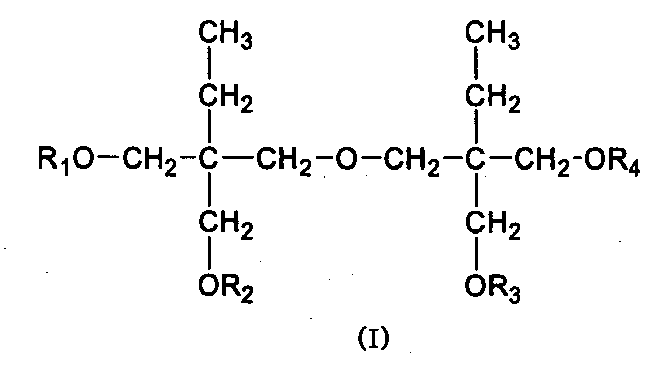 wherein R₁ to R₄ are each independently hydrogen atom or a fatty acid residue, provided that at least one of R₁ to R₄ represents a fatty acid residue.

The fatty acid for constituting the component (A) may preferably be a straight-chain or branched fatty acid having 5 to 28 carbon atoms. More preferably used are branched fatty acids. Examples of those branched fatty acids are pivalic acid, isoheptanoic acid, 4-ethylpentanoic acid, isooctylic acid, 2-ethylhexanoic acid, 4,5-dimethylhexanoic acid, 4-propylpentanoic acid, isononanoic acid, 2-ethylheptanoic acid, 3,5,5-trimethylhexanoic acid, isodecanoic acid, isododecanoic acid, 2-methyldecanoic acid, 3-methyldecanoic acid, 4-methyldecanoic acid, 5-methyldecanoic acid, 6-methyldecanoic acid, 7-methyldecanoic acid, 9-methyldecanoic acid, 6-ethylnonanoic acid, 5-propyloctanoic acid, isolauric acid, 3-methylhendecanoic acid, 6-propylnonanoic acid, isotridecanoic acid, 2-methyldodecanoic acid, 3-methyldodecanoic acid, 4-methyldodecanoic acid, 5-methyldodecanoic acid, 11-methyldodecanoic acid, 7-propyldecanoic acid, isomyristic acid, 2-methyltridecanoic acid, 12-methyltridecanoic acid, isopalmitic acid, 2-hexyldecanoic acid, 14-methylpentadecanoic acid, 2-ethyltetradecanoic acid, isostearic acid, methyl-branched isostearic acid, 2-heptylundecanoic acid, 2-isoheptylisoundecanoic acid, 2-ethylhexadecanoic acid, 14-ethylhexadecanoic acid, 14-methylheptadecanoic acid, 15-methylheptadecanoic acid, 16-methylheptadecanoic acid, 2-butyltetradecanoic acid, isoarachic acid, 3-methylnonadecanoic acid, 2-ethyloctadecanoic acid, isohexacosanoic acid, 24-methylheptacosanoic acid, 2-ethyltetracosanoic acid, 2-butyldocosanoic acid, 2-hexylicosanoic acid, 2-octyloctadecanoic acid and 2-decylhexadecanoic acid. Those fatty acids can be used alone or in combination. Among those fatty acids, preferred are fatty acids having 8 to 18 carbon atoms, in particular, branched saturated fatty acids having 8 to 18 carbon atoms, such as isooctylic acid (preferably, 2-ethylhexanoic acid and 4,5-dimethylhexanoic acid), isononanoic acid (preferably, 2-ethylheptanoic acid and 3,5,5-trimethylhexanoic acid), isopalmitic acid, isotridecanoic acid, isostearic acid (preferably, methyl-branched isostearic acid, 2-heptylundecanoic acid and 2-isoheptylisoundecanoic acid), and the like. Of those fatty acids, especially preferred are isooctylic acid, more preferably 2-ethylhexanoic acid, and isostearic acid, more preferably methyl-branched isostearic acid.

With respect to the straight-chain fatty acids, there can be employed straight-chain saturated fatty acids having 6 to 28 carbon atoms including saturated fatty acids such as caproic acid, caprylic acid, octylic acid, nonylic acid, decanoic acid, dodecanoic acid, lauric acid, tridecanoic acid, myristic acid, palmitic acid, stearic acid, behenic acid and the like; and straight-chain unsaturated fatty acids such as caproleic acid, undecylenic acid, myristoleic acid, palmitoleic acid, oleic acid, elaidic acid, gondoic acid, erucic acid, brassic acid and the like. Those fatty acids can be used alone or in combination.

The ester compound as the component (A) in the present invention includes at least one ester compound selected from mono-, di-, tri- and tetra-ester compounds. The polycarboxylic acids used to prepare the polycondensates, serving as the component (A) in the present invention, preferably include dibasic carboxylic acids having 2 to 10 carbon atoms such as succinic acid, adipic acid, azelaic acid, sebacic acid and the like. Especially, sebacic acid is preferred. Those polycarboxylic acids can be used alone or in combination.

The component (A) for use in the present invention preferably has a hydroxyl value (hereinafter referred to as "OHV") ranging from 10 to 150, more preferably 30 to 150, further more preferably 40 to 100, and most preferably 85 to 95. When a mixture of the compounds above specified is used as the component (A), it is preferable that the total OHV of the mixture be within the above-mentioned range. When the OHV is within the above-mentioned range, the hydration tendency is improved to easily offer a moisturizing feel. The term OHV herein used is a value determined by the hydroxyl value measurement test method in accordance with the Japanese Standards of Cosmetic Ingredients. Preferably, the component (A) for use in the present invention may assume a liquid state at room temperature, preferably having a viscosity of 100 to 30,000 mPa·s, more preferably 300 to 5,000 mPa·s when measured with a Brookfield viscometer at 25°C.

The component (A) for use in the present invention can be prepared, for example, by adding 1.5 to 3.5 equivalents of a fatty acid and/or polycarboxylic acid to one equivalent of ditrimethylolpropane, and carrying out a reaction of esterification and/or dehydration condensation at 180 to 240°C for 6 to 40 hours in the absence or presence of a catalyst (e.g., tin chloride). After completion of the reaction, the catalyst is removed from the reaction mixture by adsorption treatment or the like, and low-molecular weight components including an unreacted raw material are eliminated by distillation or the like, thereby obtaining a final product.

The content of the component (A) in the oil-in-water emulsion type cosmetic preparation of the present invention is not particularly limited, but may preferably be in the range of 0.1 to 30% by mass, more preferably 1 to 10% by mass, with respect to the total mass of the oil-in-water emulsion type cosmetic preparation. When the content of the component (A) is within the above-mentioned range, it becomes possible to obtain excellent products in terms of the long-lasting moisturizing feel and ease of handling.

In the present invention, it is preferable that the ester compound represented by formula (I) and/or the polycondensate of ditrimethylolpropane, fatty acid and polycarboxylic acid be contained in the component (A) in an amount of 2% by mass or more and 30% by mass or less, more preferably 8% by mass or less, and further preferably 5% by mass or less.

Any nonionic surfactants with an HLB of 10 or more and any ionic surfactants with an HLB of 10 or more that are conventionally used for the cosmetics can be employed for the component (B) in the oil-in-water emulsion type cosmetic preparation of the present invention. Specific examples of the nonionic surfactant with an HLB of 10 or more are polyglycerol fatty acid esters, polyoxyethylene hardened castor oil derivatives, polyoxyethylene castor oil derivatives; polyoxyethylene sorbitan fatty acid esters such as polyoxyethylene sorbitan monostearate, polyoxyethylene sorbitan monostearate, polyoxyethylene sorbitan monooleate, polyoxyethylene sorbitan monooleate, polyoxyethylene sorbitan trioleate and the like, polyoxyethylene glycerol fatty acid esters such as polyoxyethylene glycerol monooleate, polyoxyethylene trioleate, polyoxyethylene monoisostearate and the like; polyethylene glycol fatty acid esters such as polyethylene glycol monoisostearate; polyoxyethylene addition type nonionic surfactants such as polyoxyethylene alkyl ether, polyoxypropylene alkyl ether, polyoxyethylene alkylphenyl ether, polyoxyethylene phytostanol ether, polyoxyethylene phytosterol ether, polyoxyethylene cholestanol ether, polyoxyethylene cholesteryl ether, polyoxyalkylene-modified organopolysiloxane, and the like. Of those nonionic surfactants, preferably used are those with an HLB of 10 to 15, in particular, polyoxyethylene sorbitan fatty acid esters, more preferably polyoxyethylene sorbitan monostearate and polyoxyethylene sorbitan trioleate.

The ionic surfactant with an HLB of 10 or more includes anionic surfactants, amphoteric surfactants and cationic surfactants. Examples of the anionic surfactants are fatty acids such as stearic acid, lauric acid and the like, and soaps thereof, alkylphosphates, polyoxyethylene alkyl ether phosphates, alkyl sulfates, polyoxyethylene alkyl ether phosphates and the like. When the fatty acid soap is used as the anionic surfactant, sodium salt or potassium salt of fatty acid prepared in advance may be used. Alternatively, an alkali such as sodium hydroxide or potassium hydroxide may be mixed with a fatty acid in the preparation of the cosmetic formulation. The amphoteric surfactant includes acetate betaine, alkylaminoacetate betaine, imidazolinium betaine, and the like. The cationic surfactant includes alkylammonium salts, alkylbenzyl ammonium salts and the like. The nonionic surfactants with an HLB of less than 10 including sorbitan tristearate, sorbitan monooleate, polyoxyethylene sorbitan sesquioleate and the like can be used as an emulsifying aid or a dispersant for a powder material if added to such an extent that the effects of the present invention are not impaired.

The content of the component (B) in the oil-in-water emulsion type cosmetic preparation of the present invention is not particularly limited, but may preferably be in the range of 0.01 to 10% by mass, more preferably 0.1 to 2% by mass, with respect to the total mass of the oil-in-water emulsion type cosmetic preparation. When the content of the component (B) is within the above-mentioned range, there can be obtained the cosmetic preparations that can smoothly spread to ensure the ease of use and exhibit excellent emulsion stability over an extended period of time.

The water-based ingredient used as the component (C) in the oil-in-water emulsion type cosmetic preparation of the present invention includes water and water-soluble substances that can serve as solvents. For example, there can be employed lower alcohols such as ethyl alcohol, butyl alcohol and the like; glycols such as propylene glycol, 1,3-butylene glycol, dipropylene glycol, polyethylene glycol and the like; glycerols such as glycerin, diglycerin, polyglycerin and the like; and aqueous extracts from plants such as aloe vera, witch hazel, hamamelis, cucumber, lemon, lavender, rose and the like. One kind of water-based ingredient or two or more kinds of water-based ingredients may be selected from the above-mentioned group. It is preferable to use at least one water-based ingredient selected from the group consisting of, water, ethyl alcohol, 1,3-butylene glycol, dipropylene glycol and glycerin.

The content of the component (C) in the oil-in-water emulsion type cosmetic preparation of the present invention is not particularly limited, but may preferably be in the range of 40 to 95% by mass, more preferably 70 to 90% by mass, with respect to the total mass of the oil-in-water emulsion type cosmetic preparation. When the content of the component (C) is within the above-mentioned range, excellent products can be obtained in terms of the ease of spreading, i.e., ease of use, and the non-sticky feel. In addition, it is preferable that the component (C) contain 50% by mass or more of water.

In addition to the above-mentioned essential ingredients, various ingredients conventionally incorporated into the formulation for the cosmetic preparations, for example, an oil ingredient other than the component (A), a powder material, a water-soluble polymer, a moisturizing agent, a UV absorber, an antioxidant, a preservative, enzymes including lipase, protease and the like, pharmaceutical drugs including resorcin, sulfur and the like, a refrigerant, a coloring agent, a perfume and the like may be blended into the oil-in-water emulsion type cosmetic preparation of the present invention so far as the effects of the present invention will not be damaged.

Regardless of origin of oil, i.e., whether the oil ingredient is from animal oils, vegetable oils, synthetic oils or the like, and regardless of properties of oil, i.e., whether the oil ingredient is a solid oil, semi-solid oil, liquid oil, volatile oil or the like, the oil ingredient other than the component (A) may include hydrocarbons, fats and oils, waxes, hardened oils, ester oils, fatty acids, higher alcohols, silicone oils, fluorinated oils, oil gelling agents and the like. More specifically, there can be employed the hydrocarbons such as liquid paraffin, squalane, vaseline, polyisobutylene, polybutene, montan wax, polyethylene wax, carnauba wax, microcrystalline wax, Fischer-Tropsch wax, paraffin wax and the like; amino acid ester oils such as di(cholesteryl/behenyl/octyldodecyl) N-lauroyl-L-glutamate, di(phytosterol/behenyl/octyldodecyl) N-lauroyl-L-glutamate and the like; polyhydric alcohol fatty acid esters such as butyrospermum parkii (shea butter), cholesterol derivatives and phytosterol derivatives, such as hexaglycerin fatty acid ester, decaglycerin fatty acid ester, glyceryl oligoester (adipate/2-ethylhexanoate/stearate), dipentaerythrityl 12-hydroxystearate, cholesterol, cholestanol, dehydrocholesterol, cholesteryl lanolate, cholesteryl isostearate, cholesteryl 12-hydroxystearate, cholesteryl ricinoleate, cholesteryl macadamiate and the like; lanolin derivatives and polyoxyalkylene-modified lanolin oils, such as lanolin anhydrous, super-refined lanolin, liquid lanolin, reduced lanolin, liquid lanolin acetate, lanolin alcohol, hydrogenated lanolin alcohol, lanolin fatty acid and the like; fats and oils, such as Japan wax, olive oil, castor oil, mink oil, macadamia nut oil and the like; waxes such as beeswax, spermaceti, carnauba wax, candelilla wax and the like; esters such as jojoba oil, isopropyl myristate, isopropyl palmitate, octyldodecyl myristate, glyceryl tri-(2-ethylhexanoate), polyglyceryl diisostearate, diisostearyl malate, pentaerythrityl rosin, neopentyl glycol dioctanoate, neopentyl glycol dicaprate and the like; fatty acids such as stearic acid, lauric acid, myristic acid, behenic acid, isostearic acid, oleic acid and the like; higher alcohols such as stearyl alcohol, cetyl alcohol, lauryl alcohol, oleyl alcohol, isostearyl alcohol, behenyl alcohol and the like; silicones such as methyl polysiloxane, methylphenyl polysiloxane, decamethylcyclopentasiloxane, octamethylcyclotetrasiloxane, trimethylsiloxy silicate, methyl polysiloxane with high degree of polymerization, methylphenyl polysiloxane with high degree of polymerization, dimethyl polysiloxane with high degree of polymerization, alkoxy-modified polysiloxane and the like; fluorinated oils such as perfluorodecane, perfluorooctane, perfluoropolyether and the like; and oil gelling agents such as dextrin fatty acid esters, sucrose fatty acid esters, starch fatty acid esters, potassium stearate, 12-hydroxystearic acid and the like. Those may be used alone or in combination.

The powder material may include any powders generally used for cosmetics. Inorganic powders, optical powders, organic powders, pigment powders, metallic powders, composite powders and the like can be used without any limitation on the shape thereof, i.e., whether they may be spheres, plates, needles or the like, the particle diameter thereof, i.e., whether they may be aerosol particles, fine particles, pigment-grade particles or the like, and the particle structure thereof, i.e., whether they may be porous, non-porous or the like. Specific examples of the powder materials are inorganic white pigments such as titanium oxide, silicone-treated titanium oxide, zinc oxide, cerium oxide, barium sulfate and the like; inorganic colored pigments such as iron oxide, iron oxide black, iron oxide red, iron oxide yellow, silicone-treated iron red, carbon black, sintered material of titanium and titanium oxide, chromium oxide, chromium hydroxide, iron blue, ultramarine and the like; white extender pigments such as talc, silicone-treated talc, muscovite, phlogopite, lepidolite, biotite, synthetic mica, sericite, synthetic sericite, kaolin, silicon carbide, bentonite, smectite, silica, aluminum oxide, magnesium oxide, zirconium oxide, antimony oxide, diatomite, aluminum silicate, aluminum magnesium metasilicate, calcium silicate, barium silicate, magnesium silicate, calcium carbonate, magnesium carbonate, hydroxyapatite, boron nitride and the like; optical powders such as titanium dioxide-coated mica, titanium dioxide-coated bismuth oxychloride, iron oxide-coated titanated mica, iron blue-coated titanated mica, carmine-treated titanated mica, silicone-treated titanated mica, bismuth oxychloride, fish scale flakes, laminated powder of epoxy resin coated polyethylene terephthalate · aluminum, laminated film powder of polyethylene terephthalate · polyolefin, spherical PMMA powder, laminated film powder of polyethylene terephthalate · polymethyl methacrylate and the like; organic high-molecular weight resin powders such as polyamide resin, polyethylene resin, polyacrylic resin, polyester resin, fluoroplastic, cellulose resin, polystyrene resin, copolymer resin including styrene - acryl copolymer resin, polypropylene resin, silicone resin, urethane resin and the like; organic low-molecular weight powders such as zinc stearate, N-acyl-lysine and the like; organic natural powders such as starch, silk powder, cellulose powder and the like; organic pigment powders such as Red No. 201, Red No. 202, Red No. 205, Red No. 226, Red No. 228, Orange No. 203, Orange No. 204, Blue No. 404, Yellow No. 401 and the like; organic pigment powders of zirconium, barium or aluminum lake, such as Red No. 3, Red No. 104, Red No. 106, Orange No. 205, Yellow No. 4, Yellow No. 5, Green No. 3, Blue No.1 and the like; metallic powders such as mica, aluminum powder, gold powder, silver powder and the like; composite powders such as titanium oxide fine particles-coated titanated mica, zinc oxide fine particles-coated titanated mica, barium sulfate-coated titanated mica, titanium oxide-containing silicon dioxide, zinc oxide-containing silicon dioxide and the like. Those powder materials may be used alone or in combination, and another composite powders made from the above powders can also be used.

The UV absorber includes, for example, benzophenone compounds, PABA compounds, cinnamates, salicylates, 4-tert-butyl-4'-methoxydibenzoylmethane, oxybenzone and the like.

Examples of the water-soluble polymer include natural polymers such as xanthan gum, guar gum, sodium chondroitin sulfate, sodium hyaluronate, gum arabic, sodium alginate, carrageenan, water-swelling clay minerals and the like; semi-synthetic polymers such as methyl cellulose, hydroxyethyl cellulose, carboxymethyl cellulose and the like; and synthetic polymers such as carboxyvinyl polymer, alkyl-added carboxyvinyl polymer, polyvinyl alcohol, polyvinylpyrrolidone, sodium polyacrylate and the like. Examples of the moisturizing agent include protein, mucopolysaccharide, collagen, elastin, keratin, triethanolamine and the like.

The antioxidant includes, for example, alpha-tocopherol, ascorbic acid, magnesium L-ascorbate phosphate and the like. The beauty ingredient includes, for example, vitamins, anti-inflammatory agents, crude drugs and the like, and the preservative includes, for example, p-hydroxybenzoate, paraben, phenoxyethanol and the like.

Particularly preferable formulation examples according to the present invention will be shown below.

### Formulation Example 1

An oil-in-water emulsion type cosmetic preparation comprising:
the component (A) with an OHV of 85 to 95: an ester compound represented by formula (I), a polycondensate of ditrimethylolpropane, fatty acid and polycarboxylic acid, or a mixture thereof;
the component (B): a polyoxyethylene sorbitan fatty acid ester with an HLB of 10 to 15; and
the component (C): at least one water-based ingredient selected from the group consisting of water, ethyl alcohol, 1,3-butylene glycol, dipropylene glycol and glycerin.

### Formulation Example 2

An oil-in-water emulsion type cosmetic preparation comprising:
the component (A): an ester compound of ditrimethylolpropane and 2-ethylhexanoic acid, a polycondensate of ditrimethylolpropane, isostearic acid and sebacic acid, or a mixture thereof;
the component (B): polyoxyethylene sorbitan monostearate or polyoxyethylene sorbitan trioleate; and
the component (C): at least one water-based ingredient selected from the group consisting of water, ethyl alcohol, 1,3-butylene glycol, dipropylene glycol and glycerin.

The process for manufacturing the oil-in-water emulsion type cosmetic preparation of the present invention is not particularly limited. For example, the cosmetic preparation can be obtained in such a manner that the component (A) and other oily ingredients, if necessary, are heated and added to the component (C) and other ingredients which are also heated, and both are emulsified to form a mixture in the presence of the component (B), and then cooled.

The oil-in-water emulsion type cosmetic preparation of the present invention may be made into any product form, for example, basic skin care preparations for face, hands, feet and body, such as lotion, sunscreen, emulsion, cream, essence, cosmetic liquid for a non-woven fabric mask which is impregnated therewith, face pack, hairdressing, hair tonic and the like; and make-up preparations such as lipstick, liquid rouge, gloss, foundation, eye-shadow, base cream and the like.

The present invention will now be explained in detail by referring to the following examples, which are not intended to be limiting of the present invention.

### Examples

### Preparation Example 1

### Preparation of polycondensate of ditrimethylolpropane, isostearic acid and sebacic acid

A four-necked flask (1 L) equipped with a stirrer, a thermometer, a nitrogen gas inlet, and a reflux condenser was charged with 168 g (0.8 mol) of ditrimethylolpropane (ditrimethylolpropane made by Koei Chemical Co., Ltd.), 392 g (1.3 mol) of isostearic acid ("Prisorine 3505" made by Uniquema), and 41 g (0.2 mol) of sebacic acid (sebacic acid made by Kokura Synthetic Industries, Ltd.). Xylol was also added as a solvent for reflux in an amount of 5% by mass of the total mass of the charged materials. The mixture was allowed to react at 180 to 240°C for 6 hours with stirring. After completion of the reaction, the mixture was decolorized with activated clay and deodorized by the conventional method, so that 436 g of a polycondensate of ditrimethylolpropane, isostearic acid and sebacic acid was obtained. The OHV of the polycondensate was 92. The viscosity was 1840 mPa·s when measured with a Brookfield viscometer (25°C).

### Preparation Example 2

### Preparation of ester compound of ditrimethylolpropane and 2-ethylhexanoic acid

A four-necked flask (1 L) equipped with a stirrer, a thermometer, a nitrogen gas inlet, and a reflux condenser was charged with 211 g (0.8 mol) of ditrimethylolpropane (ditrimethylolpropane made by Koei Chemical Co., Ltd.) and 389 g (2.7 mol) of 2-ethylhexanoic acid (octylic acid made by Chisso Corporation). Xylol was also added as a solvent for reflux in an amount of 5% by mass of the total mass of the charged materials. The mixture was allowed to react at 180 to 240°C for 19 hours with stirring. After completion of the reaction, the mixture was decolorized with activated clay and deodorized by the conventional method, so that 421 g of an ester compound of ditrimethylolpropane and 2-ethylhexanoic acid was obtained. The OHV of the ester compound was 89. The viscosity was 345 mPa·s when measured with a Brookfield viscometer (25°C).

### Comparative Preparation Example 1

### Preparation of tetra-ester compound of ditrimethylolpropane, 2-ethylhexanoic acid and isostearic acid

A four-necked flask (1 L) equipped with a stirrer, a thermometer, a nitrogen gas inlet, and a reflux condenser was charged with 132 g (0.5 mol) of ditrimethylolpropane (ditrimethylolpropane made by Koei Chemical Co., Ltd.), 160 g (1.1 mol) of 2-ethylhexanoic acid (octylic acid made by Chisso Corporation) and 308 g (1.1 mol) of isostearic acid ("Prisorine 3505" made by Uniquema). Xylol was also added as a solvent for reflux in an amount of 5% by mass of the total mass of the charged materials. The mixture was allowed to react at 180 to 240°C for 30 hours with stirring. After completion of the reaction, the mixture was decolorized with activated clay and deodorized by the conventional method, so that 390 g of a tetra-ester compound of ditrimethylolpropane, 2-ethylhexanoic acid and isostearic acid (as represented by the following formula II) was obtained. The OHV of the tetra-ester compound was 1. 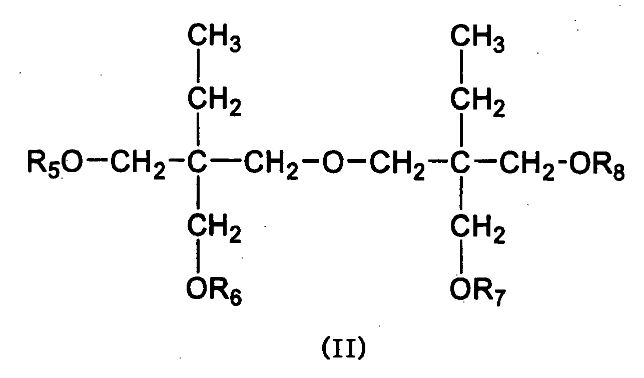 wherein R₅ to R₈ are each a 2-ethylhexanoic acid residue or isostearic acid residue, with a ratio of 2-ethylhexanoic acid residue to isostearic acid residue being 1:1.

### Examples 1 to 9 and Comparative Examples 1 to 3 (Essence)

Essences with the formulations as shown in the following Table 1 were produced by the method shown below. The skin feel (1) (a non-sticky feel and a long-lasting moisturizing feel), and the long-term emulsion stability (2) of each essence were evaluated in accordance with the manner to be described later. The results are also shown in Table 1.

**Table 1 (unit: % by mass)**

| No. | Ingredients | Examples | | | | | | | | |
|---|---|---|---|---|---|---|---|---|---|---|
| | | 1 | 2 | 3 | 4 | 5 | 6 | 7 | 8 | 9 |
| 1 | Polyoxyethylene sorbitan monooleate (20E.O.)(HLB:15) | 0.5 | 0.01 | 10 | 0.5 | 0.5 | 0.5 | 0.5 | 2 | 0.5 |
| 2 | Sorbitan esquioleate (HLB:3.7) | 0.1 | 0.01 | 0.1 | 0.1 | 0.1 | - | 0.1 | 0.1 | 0.1 |
| 3 | Polycondensate of Prep. Ex. 1 | 2 | 2 | 2 | 0.1 | 30 | 2 | 1 | 8 | 2 |
| 4 | Ester compound of Prep. Ex. 2 | - | - | - | - | - | - | 1 | - | - |
| 5 | Tetraester compound of Comp. Prep. Ex. 1 | - | - | - | - | - | - | - | - | - |
| 6 | Cetyl 2-ethyl-hexanoate | 5 | 5 | 5 | 5 | 5 | 5 | 5 | 5 | 5 |
| 7 | Ethyl alcohol | 10 | 10 | 10 | 10 | 10 | 10 | 10 | 10 | 10 |
| 8 | Perfume | 0.1 | 0.1 | 0.1 | 0.1 | 0.1 | 0.1 | 0.1 | 0.1 | 0.1 |
| 9 | Dipropylene glycol | 10 | 10 | 10 | 10 | 10 | 10 | - | 10 | 20 |
| 10 | Glycerin | 10 | 10 | 10 | 10 | 10 | 10 | 20 | 10 | - |
| 11 | Preservative | 0.1 | 0.1 | 0.1 | 0.1 | 0.1 | 0.1 | 0.1 | 0.1 | 0.1 |
| 12 | Purified water | 62.2 | 62.78 | 52.7 | 64.1 | 34.2 | 62.3 | 62.2 | 54.7 | 62.2 |
| Total | | 100 | 100 | 100 | 100 | 100 | 100 | 100 | 100 | 100 |
| Evaluation items &Results | | | | | | | | | | |
| Non-sticky skin feel | | ⊚ | ⊚ | ○ | ⊚ | ○ | ⊚ | ○ | ○ | ○ |
| Long-lasting moisturizing feel | | ⊚ | ⊚ | ⊚ | ○ | ⊚ | ⊚ | ⊚ | ⊚ | ⊚ |
| Long-term emulsion stability | | ⊚ | ○ | ⊚ | ⊚ | ○ | ○ | ⊚ | ⊚ | ⊚ |

| No. | Ingredients | Comp. Ex. | | |
|---|---|---|---|---|
| | | 1 | 2 | 3 |
| 1 | Polyoxyethylene sorbitan monooleate (20E.O.) (HLB:15) | - | 0.5 | 0.5 |
| 2 | Sorbitan sesquioleate (HLB:3.7) | 0.1 | 0.1 | 0.1 |
| 3 | Polycondensate of Prep.Ex. 1 | 2 | - | - |
| 4 | Ester compound of Prep. Ex. 2 | - | - | - |
| 5 | Tetraester compound of Comp. Prep. Ex. 1 | - | - | 2 |
| 6 | Cetyl 2-ethylhexanoate | 5 | 5 | 5 |
| 7 | Ethyl alcohol | 10 | 10 | 10 |
| 8 | Perfume | 0.1 | 0.1 | 0.1 |
| 9 | Dipropylene glycol | 10 | 10 | 10 |
| 10 | Glycerin | 10 | 10 | 10 |
| 11 | Preservative | 0.1 | 0.1 | 0.1 |
| 12 | Purified water | 62.7 | 64.2 | 62.2 |
| Total | | 100 | 100 | 100 |
| Evaluation items & Results | | | | |
| Non-sticky skin feel | | Δ | Δ | Δ |
| Long-lasting moisturizing feel | | Δ | × | Δ |
| Long-term emulsion stability | | × | ○ | × |

### (Manufacturing process)

Step A: The ingredients 1 through 6 were mixed and fused at 80°C.
Step B: The ingredients 7 through 12 were heated to 80°C and added to the mixture obtained in the step A, and the resultant mixture was emulsified.
Step C: The emulsified product obtained in the step B was cooled to obtain an essence.

### (Evaluation method for skin feel)

To evaluate a non-sticky feel and a long-lasting moisturizing feel, a sensory evaluation test was conducted using 30 female panelists who had worn make-up for 10 years or more. Each panelist assessed each sample product on the following absolute evaluation scale (a) (a 0-to-6 scale). From the average score the product was rated on four levels according to the evaluation criteria (b) shown below. The long-lasting moisturizing feel was assessed in such a manner that each sample was applied to the lips and the moisturizing effect was evaluated after a lapse of 3 hours of daily routine.
(a) Absolute evaluation scale

| <Score> : | <Evaluation> |
|---|---|
| 6: | Excellent |
| 5: | Good |
| 4: | Fair |
| 3: | Ordinary |
| 2: | Slightly poor |
| 1: | Poor |
| 0: | Very poor |

(b) Four-level rating criteria

| <Average score>: | <Rating> | |
|---|---|---|
| More than 5: | Excellent | ⊚ |
| More than 3 and 5 or less: | Good | ○ |
| More than 1 and 3 or less: | Slightly poor | Δ |
| 1 or less: | Poor | × |

### (Evaluation method for long-term emulsion stability)

Each sample was allowed to stand in a thermostant of 50°C for one month. Then, the sample was rated according to the following four-level rating criteria (c) based on the change of appearance (separation and creaming).
(c) Four-level rating criteria

| <Degree of change> : | <Rating> |
|---|---|
| No change: | ⊚ |
| Slightly changed: | ○ |
| Substantially changed: | Δ |
| Considerably changed: | × |

As is apparent from the results shown in Table 1, the essences of the present invention obtained in Examples 1 to 9 were superior to those of Comparative Examples 1 to 3 in terms of the non-sticky skin feel, the long-lasting moisturizing feel, and the long-term emulsion stability. Specifically, the product of Comparative Example 2 not using the component (A) that is one of the element constituting the present invention was unfavorable especially in terms of the long-lasting moisturizing feel. The product of Comparative Example 1 not using the component (B) that is one of the elements constituting the present invention was unfavorable especially in terms of the long-term stability. The product of Comparative Example 3 using the tetra-ester compound instead of the component (A) that is the element constituting the present invention was unfavorable especially in terms of the long-term emulsion stability.

### Example 10: Emulsion

| (Ingredients) | (% by mass) |
|---|---|
| 1. Sorbitan monooleate (HLB: 4.3) | 0.1 |
| 2. Polyoxyethylene sorbitan monostearate (20E.O.) (HLB: 14.9) | 2.0 |
| 3. Polycondensate of Preparation Example 1 | 10.0 |
| 4. Perfume | 0.02 |
| 5. Glyceryl tri-2-ethylhexanoate | 1.0 |
| 6. Dipropylene glycol | 5.0 |
| 7. Glycerin | 5.0 |
| 8. Sodium alginate | 0.5 |
| 9. Carboxyvinyl polymer | 0.2 |
| 10. Preservative (Ethyl p-hydroxybenzoate) | 0.1 |
| 11. Purified water | 75.98 |
| 12. Magnesium L- ascorbate phosphate | 0.1 |
| Total | 100 |

### (Manufacturing process)

Step A: The ingredients 1 through 5 were mixed and fused at 80°C.
Step B: The ingredients 6 through 12 were heated to 80°C and added to the mixture obtained in the step A. The resultant mixture was emulsified.
Step C: The emulsified product obtained in the step B was cooled to produce an emulsion.

The emulsion of Example 10 was excellent in terms of the non-sticky skin feel and the long-lasting moisturizing feel. Also, the emulsion showed excellent stability without separation and creaming over a long period of time.

### Example 11:Eye cream

| (Ingredients) | (% by mass) |
|---|---|
| 1. Sorbitan tristearate (HLB: 2.1) | 0.05 |
| 2. Polyoxyethylene sorbitan trioleate (20E.O.) (HLB: 11.0) | 0.1 |
| 3. Di(phytosteryl/behenyl/octyldodecyl) N-lauroyl-L-glutamate | 0.5 |
| 4. Microcrystalline wax | 0.5 |
| 5. Polybutene | 1.5 |
| 6. Stearyl alcohol | 2.5 |
| 7. Ester compound of Preparation Example 2 | 1.0 |
| 8. Dimethyl polysiloxane | 0.5 |
| 9. Dipropylene glycol | 5.0 |
| 10. Glycerin | 5.0 |
| 11. Sodium alginate | 0.1 |
| 12. Preservative (Ethyl p-hydroxybenzoate) | 0.1 |
| 13. Purified water | 83.13 |
| 14. Perfume | 0.02 |
| Total | 100 |

### (Manufacturing process)

Step A: The ingredients 1 through 8 were mixed and fused at 80°C.
Step B: The ingredients 9 through 14 were heated to 80°C and added to the mixture obtained in the step A. The resultant mixture was emulsified.
Step C: The emulsified product obtained in the step B was cooled to produce an eye cream.

The eye cream of Example 11 was excellent in terms of the non-sticky skin feel and the long-lasting moisturizing feel. Also, excellent emulsion stability was obtained without separation and creaming over a long period of time.

### Example 12: Foundation

| (Ingredients) | (% by mass) |
|---|---|
| 1. Decamethylcyclopentasiloxane | 5.0 |
| 2. Organic silicone resin solution ⁽¹⁾ | 5.0 |
| 3. Polycondensate of Preparation Example 1 | 1.0 |
| 4. Neopentyl glycol dicaprate | 2.0 |
| 5. Stearic acid | 1.5 |
| 6. Sorbitan tristearate (HLB: 2.1) | 0.05 |
| 7. Polyoxyethylene sorbitan trioleate (20E.O.) (HLB:11.0) | 0.2 |
| 8. Triethanolamine | 0.5 |
| 9. Paraben | 0.3 |
| 10. Potassium hydroxide | 0.2 |
| 11. 1,3-butylene glycol | 10.0 |
| 12. Purified water | 53.3 |
| 13. Water-swelling clay mineral ⁽²⁾ | 0.8 |
| 14. Carboxymethyl cellulose | 0.1 |
| 15. Xanthan gum | 0.05 |
| 16. Talc | 5.0 |
| 17. Mica | 3.0 |
| 18. Iron oxide black | 0.3 |
| 19. Iron oxide red | 0.7 |
| 20. Iron oxide yellow | 1.0 |
| 21. Titanium oxide | 8.0 |
| 22. Titanium oxide fine particles | 2.0 |
| Total | 100 |

| | |
|---|---|
| Note | |
| (1): "KF-9021" made by Shin-Etsu Chemical Co., Ltd. | |
| (2): "Kunipia G-4" made by Kunimine Industries Co., Ltd. | |

### (Manufacturing process)

Step A: The ingredients 1 through 7 were mixed and fused at 80°C.
Step B: The ingredients 8 through 15 were heated to 80°C, where the ingredients 16 through 22 were dispersed using a homomixer.
Step C: The dispersion system obtained in the step B was added to the mixture obtained in the step A, and the resulting mixture was emulsified and then cooled, thereby obtaining a foundation.

The foundation of Example 12 was excellent in terms of the non-sticky skin feel and the long-lasting moisturizing feel. Also, the preservation stability was excellent without separation and creaming.

### Example 13: Eye-shadow

| (Ingredients) | (% by mass) |
|---|---|
| 1. Sorbitan tristearate (HLB: 2.1) | 0.05 |
| 2. Polyoxyethylene sorbitan trioleate (20E.O.) (HLB:11.0) | 0.1 |
| 3. Polycondensate of Preparation Example 1 | 5.0 |
| 4. Diisostearyl malate | 2.0 |
| 5. Stearic acid | 0.4 |
| 6. Isostearic acid | 0.5 |
| 7. Purified water | 67.15 |
| 8. Triethanolamine | 0.8 |
| 9. Polyethylene glycol (Molecular weight: 400) | 3.0 |
| 10. Ethanol | 5.0 |
| 11. Paraben | 0.5 |
| 12. Glycerin | 3.0 |
| 13. Red No. 226 | 1.0 |
| 14. Silicone-treated iron red ⁽³⁾ | 1.0 |
| 15. Silicone-treated titanium oxide ⁽³⁾ | 0.5 |
| 16. Silicone-treated titanated mica ⁽³⁾ | 5.0 |
| 17. Silicone-treated talc ⁽³⁾ | 3.0 |
| 18 Spherical PMMA powder (Average particle diameter: 10 µm) | 2.0 |
| Total | 100 |

| | |
|---|---|
| Note (3): treated with dimethyl polysiloxane (5% by mass) | |

### (Manufacturing process)

Step A: The ingredients 1 through 6 were mixed and fused at 80°C.
Step B: The ingredients 7 through 12 were heated to 80°C, where the ingredients 13 through 18 were added and dispersed using a homomixer.
Step C: The dispersion system obtained in the step B was added to the mixture obtained in the step A, and the resulting mixture was emulsified and then cooled, thereby obtaining an eye-shadow.

The eye-shadow of Example 13 was excellent in terms of the non-sticky skin feel and the long-lasting moisturizing feel. Also, the emulsion stability was excellent without separation and creaming over a long period of time.

As previously explained in detail, the oil-in-water emulsion type cosmetic preparation of the present invention is provided with excellent properties, that is, excellent skin feel (non-sticky skin feel and long-lasting moisturizing feel), and excellent long-term emulsion stability.

## Claims

1. An oil-in-water emulsion type cosmetic preparation comprising:
(A) at least one selected from the group consisting of ester compounds represented by the following formula (I) which are reaction products of ditrimethylolpropane with fatty acid, polycondensates of ditrimethylolpropane with polycarboxylic acid, polycondensates of the ester compound of the following formula (I) with polycarboxylic acid, polycondensates of fatty acid with the polycondensate of ditrimethylolpropane with polycarboxylic acid, and polycondensates of ditrimethylolpropane, fatty acid and polycarboxylic acid, 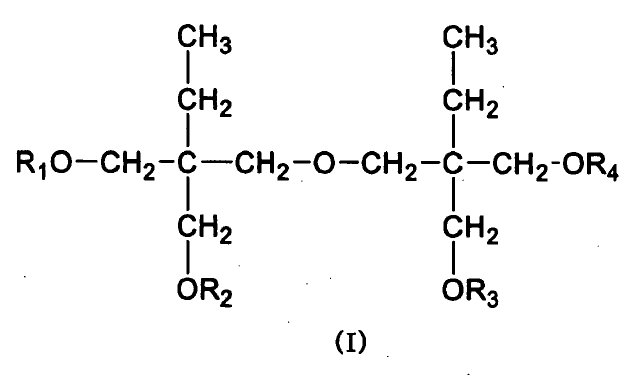
wherein R₁ to R₄ are each independently a hydrogen atom or a fatty acid residue, provided that at least one of R₁ to R₄ represents a fatty acid residue,
(B) a nonionic surfactant with an HLB of 10 or more and/or an ionic surfactant with an HLB of 10 or more; and
(C) a water-based ingredient,
wherein the component (A) has a hydroxyl value (OHV) ranging from 10 to 150.

2. The oil-in-water emulsion type cosmetic preparation as claimed in Claim 1, wherein the fatty acid residue in the formula (I) representing the component (A) is derived from a straight-chain or branched fatty acid having 5 to 28 carbon atoms.

3. The oil-in-water emulsion type cosmetic preparation as claimed in Claim 2, wherein the fatty acid residue in the formula (I) representing the component (A) is derived from the branched fatty acid.

4. The oil-in-water emulsion type cosmetic preparation as claimed in Claim 3, wherein the fatty acid residue in the formula (I) representing the component (A) is derived from the branched saturated fatty acid having 8 to 18 carbon atoms.

5. The oil-in-water emulsion type cosmetic preparation as claimed in Claim 1, wherein the polycarboxylic acid is a dibasic carboxylic acid having 2 to 10 carbon atoms.

6. The oil-in-water emulsion type cosmetic preparation as claimed in Claim 1, wherein the component (A) is obtainable by subjecting one equivalent of the ditrimethylolpropane and 1.5 to 3.5 equivalents of the fatty acid or the polycarboxylic acid or a mixture thereof to esterification and/or dehydration condensation.

7. The oil-in-water emulsion type cosmetic preparation as claimed in Claim 1, wherein the component (A) is the ester compound represented by formula (I).

8. The oil-in-water emulsion type cosmetic preparation as claimed in Claim 1, wherein the component (A) is the polycondensate of ditrimethylolpropane, fatty acid and polycarboxylic acid.

9. The oil-in-water emulsion type cosmetic preparation as claimed in Claim 1, wherein the component (A) is a mixture of the ester compound represented by formula (I) and the polycondensate of ditrimethylolpropane, fatty acid and polycarboxylic acid.

10. The oil-in-water emulsion type cosmetic preparation as claimed in any one of Claims 1 to 9, wherein the hydroxyl value (OHV) of the component (A) is in the range of 30 to 150.

11. The oil-in-water emulsion type cosmetic preparation as claimed in Claim 10, wherein the hydroxyl value (OHV) of the component (A) is in the range of 40 to 100.

12. The oil-in-water emulsion type cosmetic preparation as claimed in Claim 1, wherein the component (B) is the nonionic surfactant with an HLB of 10 to 15.

13. The oil-in-water emulsion type cosmetic preparation as claimed in Claim 12, wherein the component (B) is a polyoxyethylene sorbitan fatty acid ester.

14. The oil-in-water emulsion type cosmetic preparation as claimed in Claim 13, wherein the component (B) is polyoxyethylene sorbitan monostearate or polyoxyethylene sorbitan trioleate.

15. The oil-in-water emulsion type cosmetic preparation as claimed in Claim 1, wherein the component (C) is at least one water-based ingredient selected from the group consisting of water; lower alcohols such as ethyl alcohol and butyl alcohol; glycols such as propylene glycol, 1,3-butylene glycol, dipropylene glycol and polyethylene glycol; glycerols such as glycerin, diglycerin and polyglycerin; and aqueous extracts from plants such as aloe vera, witch hazel, hamamelis, cucumber, lemon, lavender and rose.

16. The oil-in-water emulsion type cosmetic preparation as claimed in Claim 15, wherein the component (C) is at least one water-based ingredient selected from the group consisting of water, ethyl alcohol, 1,3-butylene glycol, dipropylene glycol and glycerin.

17. The oil-in-water emulsion type cosmetic preparation as claimed in Claim 1, wherein the component (A) with an OHV of 85 to 95 is the ester compound represented by formula (I), the polycondensate of ditrimethylolpropane, fatty acid and polycarboxylic acid, or a mixture of the ester compound and the polycondensate;
the component (B) is the polyoxyethylene sorbitan fatty acid ester with an HLB of 10 to 15; and
the component (C) is at least one water-based ingredient selected from the group consisting of water, ethyl alcohol, 1,3-butylene glycol, dipropylene glycol and glycerin.

18. The oil-in-water emulsion type cosmetic preparation as claimed in Claim 17, wherein the component (A) is an ester compound of ditrimethylolpropane and 2-ethylhexanoic acid, a polycondensate of ditrimethylolpropane, isostearic acid and sebacic acid, or a mixture the ester compound and the polycondensate; and
the component (B) is polyoxyethylene sorbitan monostearate or polyoxyethylene sorbitan trioleate.

19. The oil-in-water emulsion type cosmetic preparation as claimed in any one of Claims 1 to 18, wherein the component (A) is contained in an amount of 0.1 to 30% by mass, the component (B) is contained in an amount of 0.01 to 10% by mass and the component (C) is contained in an amount of 40 to 95% by mass.

20. The oil-in-water emulsion type cosmetic preparation as claimed in Claim 19, wherein the component (A) is contained in an amount of 1 to 10% by mass, the component (B) is contained in an amount of 0.1 to 2% by mass and the component (C) is contained in an amount of 70 to 90% by mass.
